# EUROPEAN PATENT APPLICATION

(11) **EP 4 445 829 A1**
(43) Date of publication of application: **16.10.2024**
(21) Application number: 22903192.7
(22) Date of filing: 23.11.2022
(51) Int. Cl.: A61B 5/00, A61B 5/0205, A61B 5/08

(54) **WEARABLE DEVICE AND RESPIRATORY TRACT INFECTION ESTIMATION METHOD**

(30) Priority: 08.12.2021 CN 202111489780
(71) Applicant: Huawei Technologies Co., Ltd., Shenzhen, Guangdong 518129 (CN)
(72) Inventor: HOU, Zhongjie, Shenzhen, Guangdong 518129 (CN); XIONG, Hao, Shenzhen, Guangdong 518129 (CN); ZHAO, Shuai, Shenzhen, Guangdong 518129 (CN)
(74) Representative: Gill Jennings & Every LLP
(86) International application number: PCT/CN2022/133589
(87) International publication number: WO 2023/103769

(57) **Abstract**

This application provides a wearable device and a method for evaluating a respiratory tract infection. The wearable device may include a first sensor, a second sensor, and a processor. The first sensor may obtain an audio signal of a user. The second sensor may obtain a physiological parameter signal of the user. The processor may obtain a respiratory tract infection evaluation report of the user based on the physiological parameter signal and the audio signal. Based on the respiratory tract infection evaluation report, a respiratory tract infection can be detected at an early stage, to facilitate confirmation, intervention, and treatment.

## Description

This application claims priority to Chinese Patent Application No. 202111489780.7, filed with the China National Intellectual Property Administration on December 8, 2021 and entitled "WEARABLE DEVICE AND METHOD FOR EVALUATING RESPIRATORY TRACT INFECTION", which is incorporated herein by reference in its entirety.

### TECHNICAL FIELD

This application relates to the technical field of wearable devices, and specifically, to a wearable device and a method for evaluating a respiratory tract infection.

### BACKGROUND

Currently, wearable devices in the market have functions of detecting physiological parameters such as heart rate, blood oxygen, and body temperature. However, the wearable devices in the market cannot evaluate, based on the detected physiological parameters, user's risks of suffering from diseases such as a respiratory tract infection that involve a plurality of physiological parameters.

### SUMMARY

In view of this, this application provides a wearable device and a method for evaluating a respiratory tract infection, to resolve a problem that an existing wearable device cannot evaluate a user's risk of suffering from a disease.

According to a first aspect, this application provides a wearable device. The wearable device may include a first sensor, a second sensor, and a processor. The first sensor is configured to obtain an audio signal of a user. The second sensor is configured to obtain a physiological parameter signal of the user. The processor is configured to: obtain a respiratory rate of the user based on the physiological parameter signal, and obtain a respiratory tract infection evaluation report of the user based on the audio signal and the respiratory rate. It should be understood that, because the wearable device has features such as portability and portability, the wearable device helps the user perform respiratory tract infection evaluation as required. The user's risk of suffering from a respiratory tract infection can be evaluated based on the respiratory tract infection evaluation report. Moreover, the respiratory tract infection can be detected at an early stage by using the wearable device, so that confirmation, intervention, and treatment can be performed as early as possible to prevent deterioration of the respiratory tract infection.

In some implementations, the second sensor may include a PPG sensor. The PPG sensor is configured to obtain a PPG signal of the user. The processor is configured to obtain a first respiratory rate of the user based on the PPG signal. In some cases, the wearable device may obtain the respiratory tract infection evaluation report of the user based on the first respiratory rate and the audio signal.

In some implementations, the second sensor may further include an ACC sensor. The ACC sensor is configured to obtain an ACC signal of the user. The processor is configured to obtain a second respiratory rate of the user based on the ACC signal. In some cases, the wearable device may obtain the respiratory tract infection evaluation report of the user based on the second respiratory rate and the audio signal.

In some implementations, the processor is further configured to perform filtering and fusion on the first respiratory rate and the second respiratory rate, to obtain a third respiratory rate of the user. It should be understood that because the third respiratory rate is obtained based on the first respiratory rate and the second respiratory rate, accuracy of the measured respiratory rate can be improved. Based on this, accuracy of the respiratory tract infection evaluation report can be improved.

In some implementations, the processor is further configured to obtain a posture classification result of the user based on the ACC signal, where the posture classification result includes a first posture and a second posture, the first posture includes a state in which a wearing position of the user is stressed for supporting, and the second posture includes a state in which the wearing position of the user is naturally placed. It should be understood that, due to detection of a posture of the user, a corresponding respiratory rate may be used to obtain the respiratory tract infection evaluation report, thereby improving accuracy of the evaluation report.

In some implementations, when the user is in the first posture, the processor is configured to obtain the respiratory tract infection evaluation report based on the first respiratory rate and the audio signal. It should be understood that, when the user is in the first posture, the PPG sensor may obtain a PPG signal with a relatively high signal-to-noise ratio. Based on this, the first respiratory rate may be obtained based on the PPG signal, to further obtain the respiratory tract infection evaluation report.

In some implementations, when the user is in the second posture, the processor is configured to obtain the respiratory tract infection evaluation report based on the third respiratory rate and the audio signal. It should be understood that, when the user is in the second posture, the PPG sensor may obtain a PPG signal with a relatively high signal-to-noise ratio, and the ACC sensor may likewise obtain an ACC signal with a relatively high signal-to-noise ratio. Based on this, the third respiratory rate may be obtained based on the first respiratory rate and the second respiratory rate, to further obtain the respiratory tract infection evaluation report.

In some implementations, the wearable device may further include a low-pass filter. The low-pass filter is configured to perform low-pass filtering on the ACC signal. The cut-off frequency of the low-pass filter is 1 Hz. The processor is configured to: calculate a mean value and a standard deviation of the ACC signal, and calculate a power spectrum based on a low-pass filtered ACC signal, to obtain a position and an amplitude of a peak point of the power spectrum. The processor is further configured to input the mean value and the standard deviation of the ACC signal and the position and the amplitude of the peak point of the power spectrum into a classification model, to obtain the posture classification result.

In some implementations, the processor may be further configured to: when the user is in the first posture, and the first respiratory rate is beyond a preset range, prompt the user to switch to the second posture; in response to an operation performed by the user for switching to the second posture, obtain the third respiratory rate based on the PPG signal and the ACC signal; and obtain the respiratory tract infection evaluation report based on the third respiratory rate and the audio signal.

In some implementations, the wearable device may be an electronic device like a wearable watch, a wearable bracelet, or a wearable monitor. The wearable monitor may be a multiparameter monitor.

In some implementations, the wearable device may further include a first band-pass filter and a second band-pass filter. The first band-pass filter is configured to perform band-pass filtering on the PPG signal, to obtain positions of a peak point and a valley point of the PPG signal. The second band-pass filter is configured to perform band-pass filtering on the PPG signal, to obtain amplitudes of the peak point and the valley point of the PPG signal. The processor is further configured to obtain the first respiratory rate of the user based on the positions and the amplitudes of the peak point and the valley point of the PPG signal. It should be understood that, the waveform of the PPG signal can be obtained by using the second band-pass filter, and the amplitudes of the peak point and the valley point of the PPG signal can be obtained based on the positions of the peak point and the valley point of the PPG signal.

In some implementations, the frequency band of signals that are allowed to pass through the first band-pass filter is 0.5 Hz to 10 Hz, and the frequency band of signals that are allowed to pass through the second band-pass filter is 0.1 Hz to 10 Hz.

In some implementations, the wearable device may further include a third band-pass filter. The third band-pass filter is configured to perform band-pass filtering on a baseline-removed ACC signal. The processor is further configured to obtain the second respiratory rate of the user based on a band-pass filtered ACC signal.

In some implementations, the frequency band of signals that are allowed to pass through the third band-pass filter is 0.1 Hz to 0.5 Hz.

According to a second aspect, this application provides a method for evaluating a respiratory tract infection based on a wearable device. The method may include: obtaining an audio signal of a user; obtaining a physiological parameter signal of the user; obtaining a respiratory rate of the user based on the physiological parameter signal; and obtaining a respiratory tract infection evaluation report of the user based on the audio signal and the respiratory rate. It should be understood that, the user's risk of suffering from a respiratory tract infection can be evaluated based on the respiratory tract infection evaluation report. Moreover, according to the method, the respiratory tract infection can be detected at an early stage. This helps medical personnel perform confirmation, intervention, and treatment to prevent deterioration of the respiratory tract infection.

In some implementations, the obtaining a physiological parameter signal of the user specifically includes: obtaining a PPG signal of the user, where the PPG signal includes the physiological parameter signal. The obtaining a respiratory rate of the user based on the physiological parameter signal specifically includes: obtaining a first respiratory rate of the user based on the PPG signal. In some cases, the respiratory tract infection evaluation report of the user may be obtained based on the first respiratory rate and the audio signal.

In some implementations, the obtaining a physiological parameter signal of the user specifically includes: obtaining a PPG signal and an ACC signal of the user, where each of the PPG signal and the ACC signal includes the physiological parameter signal. The obtaining a respiratory rate of the user based on the physiological parameter signal specifically includes: obtaining a first respiratory rate of the user based on the PPG signal; obtaining a second respiratory rate of the user based on the ACC signal; and performing filtering and fusion on the first respiratory rate and the second respiratory rate, to obtain a third respiratory rate of the user. It should be understood that because the third respiratory rate is obtained based on the first respiratory rate and the second respiratory rate, accuracy of the measured respiratory rate can be improved. Based on this, accuracy of the respiratory tract infection evaluation report can be improved.

In some implementations, before the obtaining a physiological parameter signal of the user, the method further includes: obtaining an ACC signal of the user; and obtaining a posture classification result of the user based on the ACC signal, where the posture classification result includes a first posture and a second posture, the first posture includes a state in which a wearing position of the user is stressed for supporting, and the second posture includes a state in which the wearing position of the user is naturally placed. It should be understood that, due to detection of a posture of the user, a corresponding respiratory rate may be used to obtain the respiratory tract infection evaluation report, thereby improving accuracy of the evaluation report.

In some implementations, the obtaining a posture classification result of the user based on the ACC signal specifically includes: calculating a mean value and a standard deviation of the ACC signal; performing low-pass filtering on the ACC signal, and calculating a power spectrum of the ACC signal, to obtain a position and an amplitude of a peak point of the power spectrum, where the cut-off frequency of the low-pass filtering is 1 Hz; and inputting the mean value and the standard deviation of the ACC signal and the position and the amplitude of the peak point of the power spectrum into a classification model, to obtain the posture classification result.

In some implementations, the obtaining a respiratory tract infection evaluation report of the user based on the audio signal and the respiratory rate specifically includes: when the user is in the first posture, obtaining the respiratory tract infection evaluation report based on the first respiratory rate and the audio signal; or when the user is in the second posture, obtaining the respiratory tract infection evaluation report based on the third respiratory rate and the audio signal. It should be understood that, when the user is in the first posture, the PPG sensor may obtain a PPG signal with a relatively high signal-to-noise ratio. Based on this, the first respiratory rate may be obtained based on the PPG signal, to further obtain the respiratory tract infection evaluation report. When the user is in the second posture, in addition to the PPG signal, the ACC sensor may likewise obtain an ACC signal with a relatively high signal-to-noise ratio. Based on this, the third respiratory rate may be obtained based on the first respiratory rate and the second respiratory rate, to further obtain the respiratory tract infection evaluation report.

In some implementations, when the user is in the first posture, and the first respiratory rate is beyond a preset range, the method may further include: prompting the user to switch to the second posture; and in response to an operation performed by the user for switching to the second posture, obtaining the third respiratory rate based on the PPG signal and the ACC signal.

According to a third aspect, this application further provides a wearable device. The wearable device may include a processor, an ACC sensor, and a PPG sensor. The ACC sensor is configured to obtain an ACC signal of a user. The PPG sensor is configured to obtain a PPG signal of the user. The processor is configured to obtain a posture classification result of the user based on the ACC signal, where the posture classification result includes a first posture and a second posture, the first posture includes a state in which a wearing position of the user is stressed for supporting, and the second posture includes a state in which the wearing position of the user is naturally placed. When the user is in the first posture, the processor is further configured to obtain a first respiratory rate of the user based on the PPG signal. When the user is in the second state, the processor is further configured to: obtain a first respiratory rate of the user based on the PPG signal, obtain a second respiratory rate of the user based on the ACC signal, and perform filtering and fusion on the first respiratory rate and the second respiratory rate, to obtain a third respiratory rate of the user. It should be understood that, the wearable device can obtain the posture classification result based on detection of a posture of the user. In addition, the wearable device can obtain the respiratory rate of the user in a manner corresponding to the posture classification result, thereby improving accuracy of the measured respiratory rate.

According to a fourth aspect, this application further provides a method for measuring a respiratory rate based on a wearable device. The method may include: obtaining an ACC signal and a PPG signal that are of a user; obtaining a posture classification result of the user based on the ACC signal, where the posture classification result includes a first posture and a second posture, the first posture includes a state in which a wearing position of the user is stressed for supporting, and the second posture includes a state in which the wearing position of the user is naturally placed; and when the user is in the first posture, obtaining a first respiratory rate of the user based on the PPG signal; or when the user is in the second state, obtaining a first respiratory rate of the user based on the PPG signal, obtaining a second respiratory rate of the user based on the ACC signal, and performing filtering and fusion on the first respiratory rate and the second respiratory rate, to obtain a third respiratory rate of the user. It should be understood that, the posture classification result can be obtained based on detection of a posture of the user. In addition, the respiratory rate of the user can be obtained in a manner corresponding to the posture classification result, thereby improving accuracy of the measured respiratory rate.

According to this application, a respiratory tract infection evaluation report of a user can be obtained by obtaining an audio signal and a physiological parameter signal that are of the user, thereby implementing respiratory tract infection evaluation. Based on this, a respiratory tract infection can be detected at an early stage, to facilitate confirmation, intervention, and treatment.

### BRIEF DESCRIPTION OF DRAWINGS

FIG. 1 is a diagram of a framework of a wearable device according to an embodiment of this application;
FIG. 2 is a diagram of a framework of a wearable device according to an embodiment of this application;
FIG. 3 is a diagram of a framework of obtaining a first respiratory rate based on a PPG signal according to an embodiment of this application;
FIG. 4 is a diagram of a framework of obtaining a second respiratory rate based on an ACC signal according to an embodiment of this application;
FIG. 5 is a schematic diagram in which a user is in a first posture;
FIG. 6 is a schematic diagram in which a user is in a second posture;
FIG. 7 is a diagram of a framework of obtaining a posture classification result based on an ACC signal according to an embodiment of this application;
FIG. 8 is a schematic diagram of an interaction interface of a wearable device according to an embodiment of this application;
FIG. 9 is a flowchart of a method for evaluating a respiratory tract infection according to an embodiment of this application;
FIG. 10 is a flowchart of a method for obtaining a first respiratory rate based on a PPG signal according to an embodiment of this application;
FIG. 11 is a flowchart of a method for obtaining a first respiratory rate based on an ACC signal according to an embodiment of this application; and
FIG. 12 is a flowchart of a method for extracting a posture-related feature according to an embodiment of this application.

### DESCRIPTION OF EMBODIMENTS

Terms used in embodiments of this application are merely intended to describe specific embodiments, but are not intended to limit this application. Terms "one", "a", "the", "the foregoing", "this", and "the one" of singular forms used in this specification and the appended claims of this application are also intended to include plural forms, unless otherwise specified in the context clearly.

Reference to "an embodiment", "some embodiments", or the like described in this specification indicates that at least one embodiment of this application includes a specific feature, structure, or characteristic described with reference to the embodiment. Therefore, statements such as "in an embodiment", "in some embodiments", "in some other embodiments", "in other embodiments", and "in some implementations" that appear at different places in this specification do not necessarily mean reference to a same embodiment, but mean "one or more but not all of embodiments", unless otherwise specifically emphasized. The terms "include", "comprise", and "have", and variants thereof all mean "including but not limited to", unless otherwise specifically emphasized in another manner.

A common wearable device may be worn by a user to a wearing position of the user, to obtain physiological parameters of the user, such as heart rate, electrocardiosignal, blood oxygen saturation, and body temperature. The wearing position may be, for example, a wrist, an upper arm, or a forearm of the user. Based on this, when the user needs to view these physiological parameters, the user may view these physiological parameters by using the wearable device; or the user may view these physiological parameters by using a smart terminal that is wirelessly connected to the wearable device, where the smart terminal may be a mobile phone; or medical personnel may view these physiological parameters by using a smart terminal that is wirelessly connected to the wearable device, where the smart terminal may be a bedside monitor or a central station.

It should be understood that a common wearable device usually provides only functions of obtaining and displaying physiological parameters, but cannot evaluate, based on these physiological parameters, the user's risk of suffering from a disease.

A disease like a respiratory tract infection usually affects the physical condition of a user. For example, the user is prone to have symptoms such as cough, dyspnea, or fever. However, at an early stage of the respiratory tract infection, the physical condition of the user changes slightly, and a physiological parameter fluctuates slightly. For these changes, it is generally difficult for the user to perceive an early symptom of the respiratory tract infection, and a common wearable device cannot implement respiratory tract infection evaluation based on an obtained physiological parameter.

For the foregoing problems, the following embodiments of this application provide a wearable device and a method for evaluating a respiratory tract infection based on the wearable device. According to the wearable device and the method, a respiratory tract infection evaluation report of a user can be obtained by obtaining an audio signal and a physiological parameter signal that are of the user, and therefore, the user's risk of suffering from a respiratory tract infection can be learned. In some cases, based on the wearable device and the method provided in the following embodiments, the respiratory tract infection can be detected at an early stage, thereby moving a disease prevention and treatment gateway forward to facilitate intervention and treatment.

In some embodiments, the wearable device may be a wearable watch. In some other embodiments, the wearable device may alternatively be an electronic device like a wearable bracelet or a wearable monitor. This is not limited.

It should be understood that, in addition to implementing respiratory tract infection evaluation, the wearable device provided in the embodiments may further implement a function like tumble detection, wireless communication, or message reminding. This is not limited.

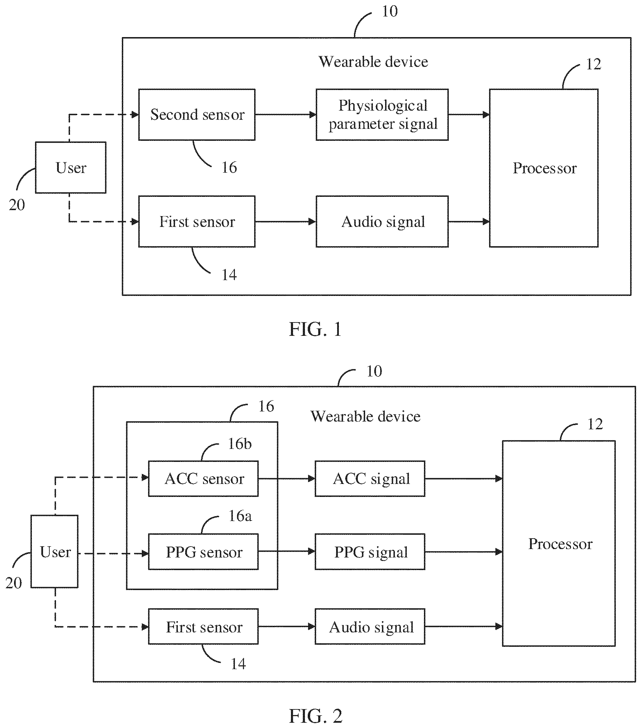

Refer to FIG. 1. A wearable device 10 provided in an embodiment of this application may include a processor 12 and a first sensor 14. The processor 12 may be electrically connected to or wirelessly connected to the first sensor 14. This is not limited. The first sensor 14 may be configured to obtain an audio signal of a user 20. The audio signal may be used to measure a sound made by the user 20 and emitted from his/her respiratory system. It should be understood that the first sensor 14 may be further configured to obtain a sound made by the user 20 within a preset time period. The preset time period may be, for example, 10 seconds, 20 seconds, 30 seconds, or 1 minute. This is not limited.

In some embodiments, the wearable device 10 may prompt the user 20 to make a cough. The first sensor 14 may measure the cough, to obtain the audio signal. Based on the audio signal, the processor 12 may perform analysis with reference to another signal, to evaluate a user 20's risk of suffering from a respiratory tract infection, and present the risk in the form of a respiratory tract infection evaluation report.

As described above, the wearable device 10 may prompt, in various manners, the user 20 to make a sound. For example, the wearable device 10 may prompt, by using a voice, the user 20 to make a cough, or may display information about prompting the user 20 to make a cough. Based on this, after the user 20 makes a cough, the wearable device 10 may respond to the cough made by the user 20, to obtain the audio signal.

In some other embodiments, a smart terminal may alternatively prompt the user 20 to make a cough in various manners, so that the first sensor 14 can collect the cough. For example, the smart terminal may prompt the user 20 by using a voice or displaying an image. After the user 20 makes a cough, the wearable device 10 may obtain the audio signal in response to the cough made by the user 20.

It should be understood that, for a user 20 without any respiratory tract infection and a user 20 with a respiratory tract infection, there is a great difference between coughs respectively made by them and emitted from their respiratory systems. The difference may be correspondingly reflected in respective audio signals. Based on this, the processor 12 may perform an operation like feature extraction on the audio signal, and classify the audio signal through machine learning or the like, thereby helping subsequently evaluate a user 20's risk of suffering from a respiratory tract infection.

In some embodiments, the processor 12 may be an MCU (Microcontroller Unit, microcontroller unit) or a CPU (Central Processing Unit, central processing unit) in the wearable device 10, but is not limited thereto. The processor 12 may alternatively be a circuit board component in the wearable device 10. The circuit board component may be integrated with an electronic component like an MCU, a CPU, a radio frequency chip, or a filter.

Still refer to FIG. 1. In some embodiments, the wearable device 10 may further include a second sensor 16. Similarly to the first sensor 14, the second sensor 16 may be electrically connected to or wirelessly connected to the processor 12. The second sensor 16 may be configured to obtain a physiological parameter signal of the user 20. The physiological parameter signal may include at least a signal used for measuring a respiratory rate of the user 20. For example, the physiological parameter signal may include a PPG signal and an ACC signal that are mentioned below, but is not limited thereto. Based on the quantity and the types of sensors provided for the wearable device 10, the physiological parameter signal may further include a signal used for measuring a physiological parameter like heart rate, electrocardiosignal, or blood oxygen saturation of the user 20.

It should be understood that, for a user 20 suffering from a respiratory tract infection, physiological parameters of the user 20 generally produce an abnormal fluctuation. The abnormal fluctuation may indicate that at least some of the physiological parameters of the user 20 are beyond a normal standard. Based on this, in the wearable device 10 provided in embodiments of this application, the processor 12 may analyze the audio signal and the physiological parameter signal of the user 20, to determine whether the physical condition of the user 20 changes. Therefore, a user 20's risk of suffering from a respiratory tract infection can be evaluated comprehensively.

In some embodiments, after respiratory tract infection evaluation is performed on the user 20, the respiratory tract infection evaluation report corresponding to the user 20 may record information such as "No abnormality is found", "The risk of suffering from a respiratory tract infection is low", "The risk of suffering from a respiratory tract infection is medium", or "The risk of suffering from a respiratory tract infection is high". It should be understood that the wearable device 10 may present a corresponding respiratory tract infection evaluation report on an interaction interface of the wearable device 10, so that the user 20 or medical personnel can view the report. Alternatively, the smart terminal may likewise present the respiratory tract infection evaluation report on an interaction interface of the smart terminal. This is not limited.

For example, for a user 20 with an early respiratory tract infection, it is relatively difficult for the user 20 to notice a body change generated when the body of the user 20 copes with the respiratory tract infection. However, the second sensor 16 of the wearable device 10 can obtain a physiological parameter signal corresponding to the body change of the user 20. The processor 12 may analyze the physiological parameter signal, to obtain a respiratory rate of the user 20. Based on this, it is learned via analysis that the respiratory rate of the user 20 is abnormal. Then, a respiratory tract infection evaluation report of the user 20 may be obtained with reference to an audio signal of the user 20. The evaluation report may record information about a high risk of a respiratory tract infection.

It should be understood that the user 20 can conveniently and quickly perform respiratory tract infection evaluation by using the wearable device 10 provided in embodiments of this application. In some cases, a respiratory tract infection can be detected at an early stage by using the wearable device 10, so that medical personnel can confirm, intervene, and treat the respiratory tract infection, to prevent deterioration of the respiratory tract infection.

Refer to FIG. 2. In some embodiments, the second sensor 16 may include a PPG (Photoplethysmography, photoplethysmography) sensor 16a. The PPG sensor 16a may obtain the PPG signal of the user 20. It should be understood that, due to commonality of human body structures, a breathing action of the user 20 have an impact on flowing of blood in a blood vessel. The impact may be correspondingly reflected in the obtained PPG signal. Based on this, the processor 12 may extract, as the physiological parameter signal from the PPG signal, a feature signal related to the breathing action of the user 20, and therefore may calculate a first respiratory rate of the user 20 based on the physiological parameter signal.

In some embodiments, the second sensor 16 may further include an ACC (Accelerometer, accelerometer) sensor 16b. The ACC sensor 16b may obtain the ACC signal of the user 20. It should be understood that, the breathing action of the user 20 may cause fluctuations of the chest cavity and the abdominal cavity of the user 20, and drive the upper limbs of the user 20 to swing. In some cases, the action of swinging the upper limbs may be captured by the ACC sensor 16b, and may be modulated in the ACC signal. Based on this, the processor 12 may extract, as the physiological parameter signal from the ACC signal, a feature signal related to the breathing action of the user 20, thereby calculating a second respiratory rate of the user 20 based on the physiological parameter signal.

In some embodiments, the PPG signal or the ACC signal may also be understood as the physiological parameter signal. The wearable device 10 may process the PPG signal and the ACC signal to obtain the respiratory rate of the user 20, but does not necessarily need to extract, as the physiological parameter signal from the PPG signal or the ACC signal, a feature signal related to the respiratory rate.

In some embodiments, there may be a plurality of first sensors, to improve accuracy of the obtained signal. In addition, there may also be a plurality of second sensors, to improve accuracy of the obtained signal.

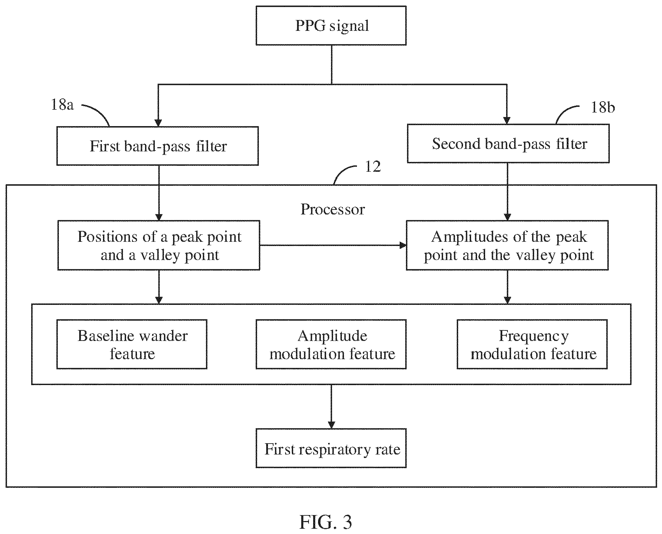

Refer to FIG. 3. In some embodiments, corresponding to the PPG signal, the wearable device 10 may further include a first band-pass filter 18a and a second band-pass filter 18b. The first band-pass filter 18a may be configured to perform band-pass filtering on the PPG signal, to obtain positions of a peak point and a valley point of the PPG signal. Similarly, the second band-pass filter 18b may be configured to perform band-pass filtering on the PPG signal, to obtain the waveform of the PPG signal.

Amplitudes of the peak point and the valley point of the PPG signal can be obtained by matching the waveform of the PPG signal with the positions of the peak point and the valley point. A baseline wander (Baseline Wander, BW) feature, an amplitude modulation (Amplitude Modulation, AM) feature, and a frequency modulation (Frequency Modulation, FM) feature that are related to the respiratory rate can be obtained based on the positions and the amplitudes of the peak point and the valley point of the PPG signal. Based on this, a first respiratory rate that is based on the PPG signal can be obtained by performing calculation on the baseline wander feature, the amplitude modulation feature, and the frequency modulation feature.

In some embodiments, the frequency band of signals that are allowed to pass through the first band-pass filter 18a may be 0.5 Hz to 10 Hz; and the frequency band of signals that are allowed to pass through the second band-pass filter 18b may be 0.1 Hz to 10 Hz.

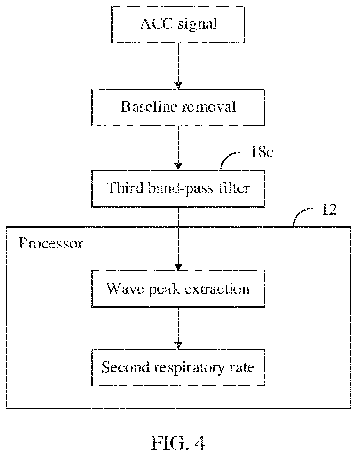

Refer to FIG. 4. In some embodiments, for the ACC signal, baseline removal may be performed on the ACC signal by using software, to prevent baseline wander interference of the ACC signal. The wearable device 10 may further include a third band-pass filter 18c. The third band-pass filter 18c may be configured to perform band-pass filtering on a baseline-removed ACC signal, to reserve a frequency band corresponding to the respiratory rate. The processor 12 may be further configured to perform wave peak extraction on a band-pass filtered ACC signal, to obtain a second respiratory rate that is based on the ACC signal.

In some embodiments, the frequency band of signals that are allowed to pass through the third band-pass filter 18c may be 0.1 Hz to 0.5 Hz.

In some embodiments, the processor 12 may be further configured to perform filtering and fusion on the first respiratory rate and the second respiratory rate, to obtain a third respiratory rate of the user 20.

It should be understood that, compared with the first respiratory rate obtained based on the PPG signal, the second respiratory rate obtained based on the ACC signal is more accurate. However, the ACC signal is also more prone to be affected by a wearing manner of the wearable device 10, a shaking action and a posture of the user 20, and the like, resulting in a more limited application scope. In some cases, the third respiratory rate that is more accurate can be obtained by performing filtering and fusion on the first respiratory rate and the second respiratory rate. Therefore, accuracy of the respiratory tract infection evaluation report can be improved.

In some embodiments, the first respiratory rate, the second respiratory rate, and the third respiratory rate may be understood as respiratory rates of the user 20 that are obtained in different manners. In the wearable device 10 provided in the embodiments, the respiratory tract infection evaluation report of the user 20 is mainly obtained based on the first respiratory rate or the third respiratory rate, but this application is not limited thereto.

In some other embodiments, the respiratory tract infection evaluation report of the user 20 may be obtained based on the second respiratory rate.

In some embodiments, the processor 12 may be further configured to obtain a posture classification result of the user 20 based on the ACC signal obtained by the ACC sensor 16b.

It should be understood that the ACC signal may include a tri-axial acceleration signal associated with the user 20. The posture classification result of the user 20 may be obtained by analyzing the ACC signal. The wearable device 10 may obtain the respiratory rate of the user 20 based on the posture classification result in different manners, to improve accuracy of the respiratory tract infection evaluation report.

The posture classification result may include a first posture and a second posture. The first posture may indicate a state in which a wearing position of the user 20 is stressed for supporting. The second posture may indicate a state in which the wearing position of the user 20 is naturally placed.

When the user 20 is in the first posture, the first respiratory rate of the user 20 may be obtained based on the PPG signal. When the user 20 is in the second posture, the third respiratory rate of the user 20 may be obtained based on the PPG signal and the ACC signal. It should be understood that the wearable device 10 may adaptively select a corresponding measurement manner based on the posture classification result. Therefore, accuracy of the measured respiratory rate can be improved.

In some embodiments, the wearable device 10 may prompt, on the interaction interface, the user 20 to perform respiratory rate measurement based on the first posture or the second posture.

In some other embodiments, the wearable device 10 may further automatically obtain the posture classification result of the user 20 based on the ACC signal. A corresponding sensor (14, 16a, or 16b) may be selected based on the posture classification result, to cooperate with the processor 12 in obtaining the respiratory rate of the user 20.

It should be understood that, when the user 20 is in a specific first posture or second posture, interference caused by an external environment and an inadvertent shake or tremor of the user 20 may be eliminated. Based on this, the wearable device 10 may obtain a PPG signal or an ACC signal with a relatively high signal-to-noise ratio, so that accuracy of the obtained first respiratory rate or third respiratory rate and accuracy of the respiratory tract evaluation report can be improved.

An example in which the wearable device 10 is a watch and the wearing position is a wrist of the user 20 is used for description.

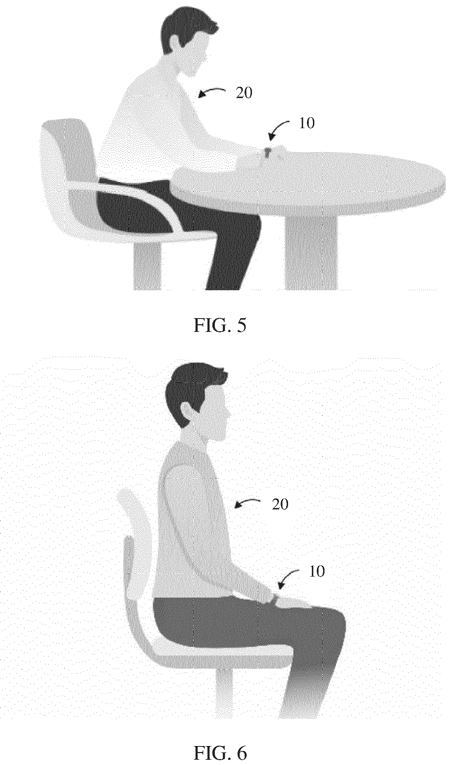

FIG. 5 illustrates a schematic diagram in which a user is in a first posture. Refer to FIG. 1 to FIG. 5 synchronously. The forearms of the user 20 that sits are stably placed on a table; and there are interaction forces between the forearms of the user 20 and the table. In this case, the PPG signal of the user 20 may be obtained based on the PPG sensor 16a. The first respiratory rate of the user 20 may be obtained by processing the PPG signal.

FIG. 6 illustrates a schematic diagram in which a user is in a second posture. Refer to FIG. 1 to FIG. 6 synchronously. The palms of the user 20 that sits are naturally placed on his thighs, and his wrists are in a naturally relaxed state. In this case, with a breathing action of the user 20, the PPG sensor 16a may obtain the PPG signal of the user 20, and the ACC sensor 16b may obtain the ACC signal of the user 20. The first respiratory rate and the second respiratory rate may be correspondingly obtained by processing the PPG signal and the ACC signal. The processor 12 may be further configured to perform filtering and fusion on the first respiratory rate and the second respiratory rate, to obtain the third respiratory rate of the user 20.

It should be understood that the first posture in FIG. 5 and the second posture in FIG. 6 are merely used as examples for description, but this application is not limited thereto. In some other embodiments, specifically, the first posture may further include a state in which the forearms of the user 20 are placed on armrests of a chair. Specifically, the second posture may further include a state in which the palms of the user 20 that sits are placed on his abdomen, or a state in which the arms of the user 20 that stands are dropped naturally.

In some embodiments, the processor 12 may extract a posture-related feature from the ACC signal, and input the extracted feature into a classification model. The classification model may classify postures of the user 20 based on these features, to distinguish between the first posture and the second posture.

In some embodiments, the classification model may include an SVM (Support Vector Machine, support vector machine), a decision tree, XGBoost, a neural network, deep learning, or the like.

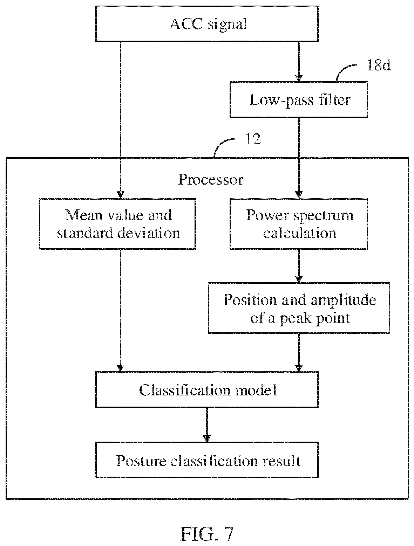

Refer to FIG. 7. In some embodiments, the processor 12 may calculate a mean value and a standard deviation of the ACC signal. In addition, the wearable device 10 may further include a low-pass filter 18d. The low-pass filter 18d is configured to perform low-pass filtering on the ACC signal. The cut-off frequency of the low-pass filter 18d is 1 Hz.

The processor 12 may further calculate a power spectrum of a low-pass filtered ACC signal, to obtain a position and an amplitude of a peak point of the power spectrum. The classification model may classify the ACC signal based on the mean value and the standard deviation of the ACC signal and the position and the amplitude of the peak point of the power spectrum, to determine a posture of the user 20 at a current moment. For example, after the ACC signal is processed, it is determined that the posture of the user 20 at the current moment is the first posture; or it is determined that the posture of the user 20 at the current moment is the second posture.

In some embodiments, the wearable device 10 may display a guide picture for the first posture or the second posture on the interaction interface of the wearable device 10, to guide the user 20 to correspondingly adjust his/her posture. In some other embodiments, the smart terminal may alternatively display the guide picture for the first posture or the second posture on the interaction interface of the smart terminal. This is not limited.

In some embodiments, when the respiratory rate (for example, the first respiratory rate or the third respiratory rate) of the user 20 is beyond a preset range, the wearable device 10 may further display prompt information on the interaction interface of the wearable device 10. The prompt information may prompt the user 20 to switch a posture and perform measurement again. If the user 20 chooses to switch the posture and perform measurement again, a signal may be obtained based on an after-switching posture by using a corresponding sensor. If the user 20 does not choose to switch the posture or actively chooses not to switch the posture, a user 20's risk of suffering from a respiratory tract infection may be evaluated based on an originally obtained respiratory rate.

In some embodiments, the prompt information of the wearable device 10 may alternatively prompt the user 20 to adjust a position of the wearable device 10 in the wearing position or re-wear the wearable device 10.

In some embodiments, the preset range of the respiratory rate may be 9 BPM to 24 BPM, where BPM is short for breaths per minute (Breaths Per Minute).

For example, when the user 20 is in the first posture, the respiratory rate of the user 20 obtained by the wearable device 10 is 8 BPM, that is, the respiratory rate is beyond the preset range of the respiratory rate. Based on this, the user 20 may be prompted on the interaction interface of the wearable device 10 to switch to the second posture and perform detection again. If the user 20 chooses to perform switching, the wearable device 10 may measure the respiratory rate of the user 20 again in response to the operation of the user 20. If the user 20 chooses not to perform switching, the wearable device 10 may obtain the respiratory tract infection evaluation report of the user 20 based on the previously obtained respiratory rate (namely, 8 BPM) with reference to the audio signal.

In some embodiments, in a process of implementing respiratory tract infection evaluation, the wearable device 10 may further provide prompt information in the form of a picture, a text, a voice, or the like, to prompt the user 20 to perform a related operation.

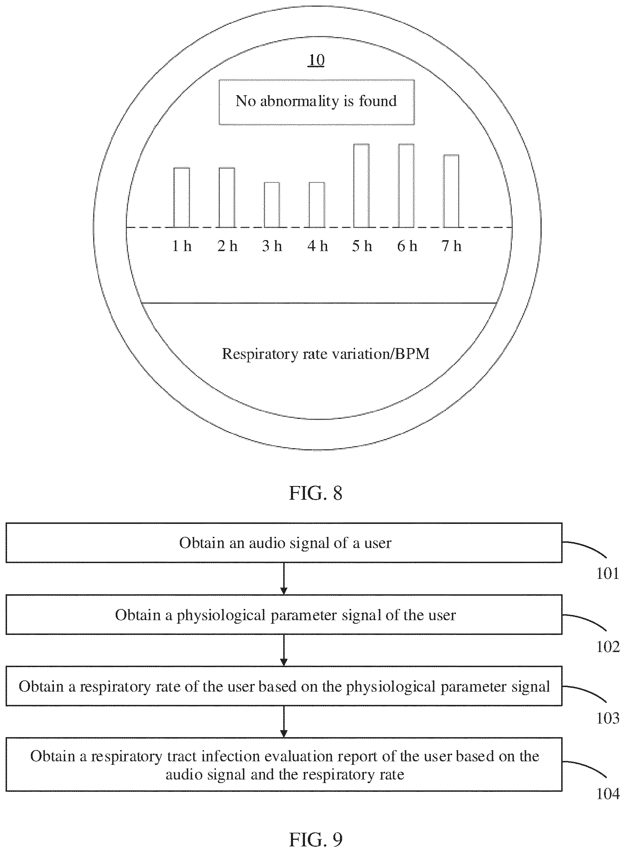

Refer to FIG. 8. In some embodiments, the wearable device 10 may display the respiratory tract infection evaluation report, and may further display information related to the respiratory rate. For example, the wearable device may illustratively display changes of the respiratory rate of the user 20 in a period of time. The period of time may be recent several minutes, tens of minutes, or several hours. As shown in FIG. 8, for example, the respiratory tract infection evaluation report shows that no abnormality is found, and the wearable device 10 further displays changes of the respiratory rate of the user 20 from 1 h to 7 h.

In some embodiments, the wearable device 10 may be further configured to measure a respiratory rate of the user 20 in daily work and life. For example, the first respiratory rate or the third respiratory rate is correspondingly obtained based on a posture of the user 20. The first respiratory rate or the third respiratory rate may be displayed in real time on a main interface or a default interface of the wearable device, so that the user 20 can view the first respiratory rate or the third respiratory rate. The main interface may be an interface defined by a manufacturer, or may be an interface that is displayed after the wearable device is powered on and that is visible to a user without requiring any operation. The default interface may be an interface that is in the wearable device and that is provided for user-defined settings, or the default interface may be the main interface.

It should be understood that, for some wearable devices, after different theme plug-ins are loaded, main interfaces of these wearable devices may display a respiratory rate or may not display a respiratory rate, but the main interfaces should still be considered as the main interface or the default interface in the foregoing embodiments.

Refer to FIG. 9. An embodiment of this application further provides a method for evaluating a respiratory tract infection based on a wearable device. Similarly to the wearable device provided in the foregoing embodiments, the method may also implement respiratory tract infection evaluation. The method may include but is not limited to the following steps.

101: Obtain an audio signal of a user.

In some embodiments, the audio signal may be used to measure a sound made by the user and emitted from his/her respiratory system. For example, the audio signal is obtained by measuring a cough made by the user. Based on the audio signal, analysis may be performed according to the method with reference to another signal, to evaluate the user's risk of suffering from a respiratory tract infection, and present the risk in the form of a respiratory tract infection evaluation report.

In some embodiments, the user may be prompted on the interaction interface of the wearable device to make a sound, for example, a cough. Then, the audio signal is obtained in response to the cough made by the user. In addition, the user may alternatively be prompted on the interaction interface of the smart terminal to make a cough; and the wearable device may obtain the audio signal in response to the cough.

It should be understood that, for a normal user (without any respiratory tract infection) and a user with a respiratory tract infection, there is a great difference between coughs respectively made by them. The difference may be correspondingly reflected in respective audio signals. Based on this, an operation like feature extraction may be performed on the audio signal; and the audio signal may be classified through machine learning or the like. This helps subsequently evaluate the user's risk of suffering from a respiratory tract infection.

102: Obtain a physiological parameter signal of the user.

The physiological parameter signal may be used to measure a respiratory rate of the user, but is not limited thereto. Based on the quantity and the types of sensors provided for the wearable device, the physiological parameter signal may be further used to measure a physiological parameter like heart rate, electrocardiosignal, or blood oxygen saturation of the user.

It should be understood that on the premise of no conflict, there is no necessary sequence between the steps. For example, for step 101 and step 102, step 101 may be performed before step 102; or, step 102 may be performed before step 101.

103: Obtain a respiratory rate of the user based on the physiological parameter signal.

The physiological parameter signal may be a part of a PPG signal or an ACC signal. The physiological parameter signal may be obtained by analyzing the PPG signal or the ACC signal. Then, the respiratory rate of the user may be obtained.

It should be understood that the PPG signal or the ACC signal may also be directly understood as the physiological parameter signal. According to the method, the PPG signal and the ACC signal may be processed to obtain the respiratory rate of the user; but a feature signal related to the respiratory rate does not necessarily need to be extracted as the physiological parameter signal from the PPG signal or the ACC signal.

104: Obtain a respiratory tract infection evaluation report of the user based on the audio signal and the respiratory rate.

In some embodiments, because the respiratory rate of the user is directly associated with the health condition of his/her respiratory system, it may be determined, by analyzing the audio signal and the respiratory rate of the user, whether the physical condition of the user changes. Then, the user's risk of suffering from a respiratory tract infection can be comprehensively evaluated.

In some embodiments, after respiratory tract infection evaluation is performed on the user, the respiratory tract infection evaluation report corresponding to the user may record information such as "No abnormality is found", "The risk of suffering from a respiratory tract infection is low", "The risk of suffering from a respiratory tract infection is medium", or "The risk of suffering from a respiratory tract infection is high". It should be understood that the respiratory tract infection evaluation report may be presented on the interaction interface of the wearable device or the smart terminal, so that the user can view the report.

For example, for a user with an early respiratory tract infection, it is relatively difficult for the user to notice a change that is of his/her physical condition and that is generated when the body of the user copes with the respiratory tract infection. According to the method provided in embodiments of this application, the respiratory rate of the user can be obtained by obtaining the physiological parameter signal corresponding to the change of the physical condition of the user and analyzing the physiological parameter signal. Based on this, it is learned via analysis that the respiratory rate of the user is abnormal. Then, the respiratory tract infection evaluation report of the user may be obtained with reference to the audio signal of the user. The evaluation report records information indicating that the user has a high risk of suffering from a respiratory tract infection.

It should be understood that according to the method provided in embodiments of this application, a user can conveniently and quickly implement respiratory tract infection evaluation by using a wearable device. In some cases, a respiratory tract infection can be detected at an early stage according to the method, so that medical personnel can confirm, intervene, and treat the respiratory tract infection, to prevent deterioration of the respiratory tract infection.

In some embodiments, the respiratory tract infection evaluation report further records information related to the respiratory rate. For example, the respiratory tract infection evaluation report may record a respiratory rate distribution condition of the user in a period of time, where the period of time may be recent several minutes, tens of minutes, or several hours.

Before step 101, the method provided in embodiments of this application may further include: detecting whether the user wears the wearable device.

In some embodiments, if the user wears the wearable device, step 101 may be performed, to implement respiratory tract infection evaluation for the user.

In some other embodiments, if the user does not wear the wearable device, step 101 is not performed. In some cases, the method may further prompt, on the interaction interface of the wearable device or the smart terminal, the user to wear the wearable device, so as to perform step 101.

In some embodiments, in the aspect of obtaining the physiological parameter signal, the method may specifically include: obtaining a PPG signal of the user.

It should be understood that a breathing-related feature signal may be extracted as the physiological parameter signal from the PPG signal, and that a first respiratory rate of the user is calculated based on the physiological parameter signal.

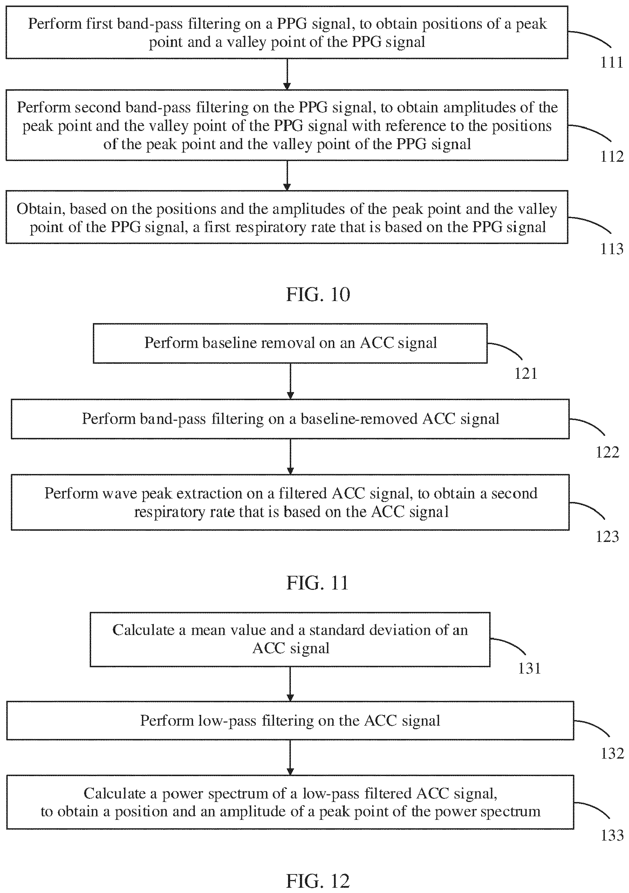

Refer to FIG. 10. In some embodiments, corresponding to the PPG signal, the method may further specifically include the following steps.

111: Perform first band-pass filtering on the PPG signal, to obtain positions of a peak point and a valley point of the PPG signal.

In some embodiments, the filtering may be performed on the PPG signal by using a first band-pass filter. The frequency band of signals that are allowed to pass through the first band-pass filter may be 0.5 Hz to 10 Hz.

112: Perform second band-pass filtering on the PPG signal, to obtain amplitudes of the peak point and the valley point of the PPG signal with reference to the positions of the peak point and the valley point of the PPG signal.

In some embodiments, the filtering may be performed on the PPG signal by using a second band-pass filter, to obtain the waveform of the PPG signal. The amplitudes of the peak point and the valley point of the PPG signal can be obtained by matching the waveform of the PPG signal with the positions of the peak point and the valley point.

In some embodiments, the frequency band of signals that are allowed to pass through the second band-pass filter may be 0.1 Hz to 10 Hz.

113: Obtain, based on the positions and the amplitudes of the peak point and the valley point of the PPG signal, a first respiratory rate that is based on the PPG signal.

In some embodiments, a baseline wander feature, an amplitude modulation feature, and a frequency modulation feature that are related to a respiratory rate may be obtained based on the positions and the amplitudes of the peak point and the valley point of the PPG signal. The first respiratory rate that is based on the PPG signal can be obtained by performing calculation on the baseline wander feature, the amplitude modulation feature, and the frequency modulation feature.

In some embodiments, in the aspect of obtaining the physiological parameter signal, the method may further specifically include: obtaining an ACC signal of the user.

It should be understood that, a breathing action of the user may cause fluctuations of the chest cavity and the abdominal cavity of the user, and drive the upper limbs of the user to swing. In some cases, the action of swinging the upper limbs may be captured by an ACC sensor, and may be modulated in the ACC signal. Based on this, a breathing-related feature signal may be extracted as the physiological parameter signal from the ACC signal; and a second respiratory rate of the user is calculated based on the physiological parameter signal.

Refer to FIG. 11. In some embodiments, for the ACC signal, the method may specifically include the following steps.

121: Perform baseline removal on the ACC signal.

Based on step 121, the ACC signal may be baseline removed through software, to prevent baseline wander interference of the ACC signal, so that accuracy of a measured respiratory rate is improved.

122: Perform band-pass filtering on a baseline-removed ACC signal.

In some embodiments, the filtering may be performed on the ACC signal by using a third band-pass filter, to reserve a frequency band that is of the ACC signal and that corresponds to the respiratory rate. The frequency band of signals that are allowed to pass through the third band-pass filter may be 0.1 Hz to 0.5 Hz.

123: Perform wave peak extraction on a filtered ACC signal, to obtain a second respiratory rate that is based on the ACC signal.

In some embodiments, in the aspect of obtaining the respiratory rate, the method may further include:
performing filtering and fusion on the first respiratory rate and the second respiratory rate, to obtain a third respiratory rate of the user.

It should be understood that, compared with the first respiratory rate obtained based on the PPG signal, the second respiratory rate obtained based on the ACC signal is more accurate. However, the ACC signal is also more prone to be affected by a wearing manner of the wearable device, a shaking action and a posture of the user, and the like, resulting in a more limited application scope. In some cases, the third respiratory rate that is more accurate can be obtained by performing filtering and fusion on the first respiratory rate and the second respiratory rate. Therefore, accuracy of the respiratory tract infection evaluation report can be improved.

In some embodiments, the first respiratory rate, the second respiratory rate, and the third respiratory rate may be understood as respiratory rates of the user that are obtained in different manners. In the method provided in the embodiments, the respiratory tract infection evaluation report of the user is mainly obtained based on the first respiratory rate or the third respiratory rate, but this application is not limited thereto. In some other embodiments, the respiratory tract infection evaluation report of the user may alternatively be obtained based on the second respiratory rate.

In some embodiments, to improve measurement accuracy of the respiratory rate, before the obtaining a physiological parameter signal, the method may further include: obtaining a posture classification result of the user based on the ACC signal.

It should be understood that the ACC signal may include a tri-axial acceleration signal associated with the user. The posture classification result of the user may be obtained by analyzing the ACC signal. The respiratory rate of the user may be obtained based on the posture classification result in different manners, to improve accuracy of the respiratory tract infection evaluation report.

In some embodiments, the posture classification result may include a first posture and a second posture. The first posture may indicate a state in which a wearing position of the user is stressed for supporting. The second posture may indicate a state in which the wearing position of the user is naturally placed.

When the user is in the first posture, the first respiratory rate of the user may be obtained based on the PPG signal. When the user is in the second posture, the third respiratory rate of the user may be obtained based on the PPG signal and the ACC signal. It should be understood that a corresponding measurement manner may be adaptively selected based on the posture classification result. Therefore, accuracy of the measured respiratory rate and accuracy of the respiratory tract infection evaluation report can be improved.

In some embodiments, similarly to prompting the user to make a sound, the user may be prompted on the interaction interface of the wearable device to perform respiratory rate measurement based on the first posture or the second posture.

In some other embodiments, the posture classification result of the user may be further obtained automatically based on the ACC signal. A corresponding method may be selected based on the posture classification result, to obtain the respiratory rate of the user.

In some embodiments, in the aspect of obtaining the posture classification result of the user based on the ACC signal, the method may specifically include: extracting a posture-related feature from the ACC signal, and inputting the extracted feature into a classification model. The classification model may classify postures of the user based on these features.

Refer to FIG. 12. In some embodiments, in the aspect of extracting the posture-related feature, the method may specifically include the following steps.

131: Calculate a mean value and a standard deviation of the ACC signal.

132: Perform low-pass filtering on the ACC signal.

In some embodiments, the filtering may be performed on the ACC signal by using a low-pass filter. The cut-off frequency of the low-pass filter may be 1 Hz.

133: Calculate a power spectrum of a low-pass filtered ACC signal, to obtain a position and an amplitude of a peak point of the power spectrum.

In some embodiments, the classification model may classify the ACC signal based on the mean value and the standard deviation of the ACC signal and the position and the amplitude of the peak point of the power spectrum, to determine a posture of the user at a current moment.

In some embodiments, a guide picture for the first posture or the second posture may be displayed on the interaction interface of the wearable device, to guide the user to correspondingly adjust his/her posture. In some other embodiments, the guide picture for the first posture or the second posture may alternatively be displayed on the interaction interface of the smart terminal. This is not limited.

In some embodiments, when the respiratory rate (for example, the first respiratory rate or the third respiratory rate) of the user is beyond a preset range, prompt information may be further displayed on the interaction interface of the wearable device. The prompt information may prompt the user to switch a posture and perform detection again. If the user chooses to switch the posture and perform measurement again, a signal may be obtained based on an after-switching posture by using a corresponding sensor. If the user does not choose to switch the posture or actively chooses not to switch the posture, the user's risk of suffering from a respiratory tract infection may be evaluated based on an originally obtained respiratory rate.

In some embodiments, the prompt information may alternatively prompt the user to adjust a position of the wearable device in the wearing position or re-wear the wearable device.

In some embodiments, the preset range of the respiratory rate may be 9 BPM to 24 BPM. For example, when the user is in the first posture, and the respiratory rate obtained based on the PPG signal is 25 BPM, that is, the respiratory rate is beyond the preset range of the respiratory rate. Based on this, the user may be prompted on the interaction interface of the wearable device to switch to the second posture and perform detection again. If the user chooses to perform switching, the respiratory rate of the user may be measured again in response to the operation of the user. If the user chooses not to perform switching, the respiratory tract infection evaluation report of the user may be obtained based on the previously obtained respiratory rate (namely, 26 BPM) with reference to the audio signal.

In some embodiments, in a process of implementing the method for evaluating a respiratory tract infection, prompt information may be further provided in the form of a picture, a text, a voice, or the like, to prompt the user to perform a related operation.

In some embodiments, the method may further include: providing guide information in the form of a picture, a text, a voice, or the like. The guide information may be used to guide the user to perform a related operation, thereby obtaining and processing the audio signal and the physiological parameter signal.

## Claims

1. A wearable device, wherein the wearable device comprises:
a first sensor, configured to obtain an audio signal of a user;
a second sensor, configured to obtain a physiological parameter signal of the user; and
a processor, configured to: obtain a respiratory rate of the user based on the physiological parameter signal, and obtain a respiratory tract infection evaluation report of the user based on the audio signal and the respiratory rate.

2. The wearable device according to claim 1, wherein the second sensor comprises a PPG sensor, the PPG sensor is configured to obtain a PPG signal of the user, and the processor is configured to obtain a first respiratory rate of the user based on the PPG signal.

3. The wearable device according to claim 2, wherein the second sensor further comprises an ACC sensor, the ACC sensor is configured to obtain an ACC signal of the user, and the processor is further configured to obtain a second respiratory rate of the user based on the ACC signal.

4. The wearable device according to claim 3, wherein the processor is further configured to perform filtering and fusion on the first respiratory rate and the second respiratory rate, to obtain a third respiratory rate of the user.

5. The wearable device according to claim 3 or 4, wherein the processor is further configured to obtain a posture classification result of the user based on the ACC signal, the posture classification result comprises a first posture and a second posture, the first posture comprises a state in which a wearing position of the user is stressed for supporting, and the second posture comprises a state in which the wearing position of the user is naturally placed;
when the user is in the first posture, the processor is configured to obtain the respiratory tract infection evaluation report based on the first respiratory rate and the audio signal; and
when the user is in the second posture, the processor is configured to obtain the respiratory tract infection evaluation report based on the third respiratory rate and the audio signal.

6. The wearable device according to claim 5, wherein the wearable device further comprises a low-pass filter, the low-pass filter is configured to perform low-pass filtering on the ACC signal, and a cut-off frequency of the low-pass filter is 1 Hz;
the processor is configured to: calculate a mean value and a standard deviation of the ACC signal, and calculate a power spectrum based on a low-pass filtered ACC signal, to obtain a position and an amplitude of a peak point of the power spectrum; and
the processor is further configured to input the mean value and the standard deviation of the ACC signal and the position and the amplitude of the peak point of the power spectrum into a classification model, to obtain the posture classification result.

7. The wearable device according to claim 5, wherein the processor is further configured to: when the user is in the first posture, and the first respiratory rate is beyond a preset range, prompt the user to switch to the second posture; and
the processor is further configured to: in response to an operation performed by the user for switching to the second posture, obtain the third respiratory rate based on the PPG signal and the ACC signal, and obtain the respiratory tract infection evaluation report based on the third respiratory rate and the audio signal.

8. The wearable device according to any one of claims 1 to 6, wherein the wearable device is a wearable watch, a wearable bracelet, or a wearable monitor.

9. A method for evaluating a respiratory tract infection based on a wearable device, wherein the method comprising:
obtaining an audio signal of a user;
obtaining a physiological parameter signal of the user;
obtaining a respiratory rate of the user based on the physiological parameter signal; and
obtaining a respiratory tract infection evaluation report of the user based on the audio signal and the respiratory rate.

10. The method according to claim 9, wherein the obtaining a physiological parameter signal of the user specifically comprises:
obtaining a PPG signal of the user, wherein the PPG signal comprises the physiological parameter signal; and
the obtaining a respiratory rate of the user based on the physiological parameter signal specifically comprises:
obtaining a first respiratory rate of the user based on the PPG signal.

11. The method according to claim 9, wherein the obtaining a physiological parameter signal of the user specifically comprises:
obtaining a PPG signal and an ACC signal of the user, wherein each of the PPG signal and the ACC signal comprises the physiological parameter signal; and
the obtaining a respiratory rate of the user based on the physiological parameter signal specifically comprises:
obtaining a first respiratory rate of the user based on the PPG signal;
obtaining a second respiratory rate of the user based on the ACC signal; and
performing filtering and fusion on the first respiratory rate and the second respiratory rate, to obtain a third respiratory rate of the user.

12. The method according to any one of claims 9 to 11, wherein before the obtaining a physiological parameter signal of the user, the method further comprises:
obtaining an ACC signal of the user; and
obtaining a posture classification result of the user based on the ACC signal, wherein
the posture classification result comprises a first posture and a second posture, the first posture comprises a state in which a wearing position of the user is stressed for supporting, and the second posture comprises a state in which the wearing position of the user is naturally placed; and
the obtaining a respiratory tract infection evaluation report of the user based on the audio signal and the respiratory rate specifically comprises:
when the user is in the first posture, obtaining the respiratory tract infection evaluation report based on the first respiratory rate and the audio signal; or
when the user is in the second posture, obtaining the respiratory tract infection evaluation report based on the third respiratory rate and the audio signal.

13. The method according to claim 11, wherein the obtaining a posture classification result of the user based on the ACC signal specifically comprises:
calculating a mean value and a standard deviation of the ACC signal;
performing low-pass filtering on the ACC signal, and calculating a power spectrum of the ACC signal, to obtain a position and an amplitude of a peak point of the power spectrum, wherein a cut-off frequency of the low-pass filtering is 1 Hz; and
inputting the mean value and the standard deviation of the ACC signal and the position and the amplitude of the peak point of the power spectrum into a classification model, to obtain the posture classification result.

14. The method according to claim 13, wherein when the user is in a first posture, and the first respiratory rate is beyond a preset range, the method further comprises:
prompting the user to switch to a second posture;
in response to an operation performed by the user for switching to the second posture, obtaining the third respiratory rate based on the PPG signal and the ACC signal; and
obtaining the respiratory tract infection evaluation report based on the third respiratory rate and the audio signal.
